Europäisches Patentamt

European Patent Office

Office européen des brevets

(19)

(11) **EP 0 884 993 B1**

(12) **EUROPÄISCHE PATENTSCHRIFT**

(45) Veröffentlichungstag und Bekanntmachung des Hinweises auf die Patenterteilung:
**31.07.2002  Patentblatt 2002/31**

(51) Int Cl.$^7$: **A61F 13/15**, A61L 15/42, A61L 15/60

(21) Anmeldenummer: **97907049.7**

(22) Anmeldetag: **27.02.1997**

(86) Internationale Anmeldenummer:
**PCT/EP97/00963**

(87) Internationale Veröffentlichungsnummer:
**WO 97/31600 (04.09.1997 Gazette 1997/38)**

(54) **ABSORBERELEMENT AUS SUPERABSORBIERENDEN SCHÄUMEN MIT ANISOTROPEM QUELLVERHALTEN**

ABSORBER ELEMENT OF SUPERABSORBENT FOAMS HAVING ANISOTROPIC SWELLING BEHAVIOUR

ELEMENT ABSORBANT COMPOSE DE MOUSSES SUPERABSORBANTES A POUVOIR DE GONFLEMENT ANISOTROPE

(84) Benannte Vertragsstaaten:
**DE ES FR GB IT SE**

(30) Priorität: **28.02.1996  DE 19607529**

(43) Veröffentlichungstag der Anmeldung:
**23.12.1998  Patentblatt 1998/52**

(73) Patentinhaber: **BASF AKTIENGESELLSCHAFT 67056 Ludwigshafen (DE)**

(72) Erfinder:
• **HÄHNLE, Hans-Joachim D-67435 Neustadt (DE)**
• **WALTER, Manfred D-67346 Speyer (DE)**
• **TROPSCH, Jürgen D-67354 Römerberg (DE)**
• **KREMESKÖTTER, Jens D-67063 Ludwigshafen (DE)**
• **SCHORNICK, Gunnar D-67271 Neuleiningen (DE)**
• **ANSTOCK, Thomas D-67256 Weisenheim (DE)**

(74) Vertreter: **Kinzebach, Werner, Dr. et al Patent Attorneys, Reitstötter, Kinzebach & Partner, Sternwartstrasse 4 81679 München (DE)**

(56) Entgegenhaltungen:
EP-A- 0 438 113          WO-A-94/01069
US-A- 5 175 046          US-A- 5 338 766

**Beschreibung**

Die vorliegende Erfindung betrifft Absorberelemente mit anisotropem Quellverhalten aus wenigstens einem Verbundmaterial mit absorbierenden Elementen auf einem Träger sowie deren Herstellung und Verwendung.

Wasserabsorbierende, schaumförmige, vernetzte Polymerisate, die häufig als Superabsorber oder superabsorbierende Polymere bzw. Schäume bezeichnet werden, sind in der Lage, ein Vielfaches ihres Eigengewichtes an wäßrigen Flüssigkeiten unter Ausbildung von Hydrogelen aufzunehmen. Diese Polymerisate werden daher beispielsweise in Hygieneprodukten, wie Windeln, zur Absorption von Urin eingesetzt. Sie haben die Eigenschaft, die absorbierte Flüssigkeit auch unter mechanischer Belastung zurückzuhalten.

Die erwähnten Polymerisate kommen in Hygiene- oder Sanitärprodukten häufig in Form von Verbundmaterialien zur Anwendung. So beschreibt die WO-A-88/09801 ein in Wasser quellbares, polymeres Absorptionsmaterial, das in Form eines Laminates mit einem Gewebe eingesetzt werden kann. Die WO-A-94/07935 beschreibt wasserabsorbierende hydrophile Polyurethangelschäume, die auf flächige Träger, wie Gewebe, Gewirke, Vliese oder Folien, aufgetragen werden können.

Aus der EP-A-427 219 und der US-A-4,990,541 sind wasserabsorbierende Latexschäume bekannt, in die nach dem Schäumprozeß superabsorbierende, feinteilige Materialien eingearbeitet werden.

Die WO-A-94/22502 (US 5,338,766) beschreibt einen superabsorbierenden Polymerschaum, der zur Herstellung von Hygieneartikeln, wie Windeln, verwendet werden kann. Zu diesem Zweck wird der Polymerschaum auf einen Träger aufgebracht, wobei der Polymerschaum die Oberfläche des Trägers zum Teil oder vollständig bedecken und in einem beliebigen Muster aufgebracht sein kann. Das Muster wird dabei aus größeren zusammenhängenden Flächen des Polymerschaums gebildet und hat den Zweck, die absorbierenden Bereiche des Hygieneartikels in der gewünschten Weise zu verteilen.

Die US 5,175,046 offenbart eine Laminatstruktur, worin diskrete Elemente eines superabsorbierenden Materials auf einer zusammenhängenden porösen Trägerschicht befestigt sind. Die superabsorbierenden Elemente werden erhalten, indem man ein poröses Substrat mit einer Acrylsäuremonomerlösung tränkt, das Polymer im Gefüge des porösen Substrats polymerisiert und vernetzt und das Substrat unter Erhalt diskreter superabsorbierender Elemente zerschneidet. Die superabsorbierenden Elemente werden dann durch Friktion auf einer flexiblen porösen Trägerschicht befestigt.

Die superabsorbierenden Polmyere besitzen die Eigenschaft, sich bei der Aufnahme von wäßrigen Flüssigkeiten im wesentlichen isotrop nach allen drei Richtungen des Raumes auszudehnen. Im allgemeinen dehnen sich die Polymere in jeder Dimension um den Faktor 2 bis 5 aus, so daß insgesamt eine Volumenzunahme um den Faktor 8 bis 125 zu beobachten ist. In der Praxis hat sich gezeigt, daß diese Eigenschaft bei der Konstruktion von Hygieneartikeln nachteilig ist, da eine flächenmäßige Ausdehnung der Polymere über einen größeren Bereich hinweg in der Regel nicht möglich ist. Außerdem ist zu berücksichtigen, daß bei einer Flächenausdehnung um den Faktor 4 bis 25 beim Einlegen einer einfachen Schaumschicht in eine Windel nur zwischen 1/4 bzw. 1/25 der Windelfläche durch den Schaum bedeckt sein dürfte, um genügend Platz für das Ausquellen zu haben. Dies hat zur Folge, daß auf diese Weise eine effektive Aufnahme der freigesetzten Körperflüssigkeiten nicht in jedem Fall gewährleistet ist, so daß eine vollständige Aaufnahme beispielsweise bei Windeln auf andere Weise sichergestellt werden muß. Wird dagegen die gesamte verfügbare Fläche in einer Windel mit einer superabsorbierenden Polymerschicht bedeckt, so ist, wie erwähnt, nicht genügend Platz zur Verfügung, um ein effektives und rasches Ausquellen der Polymerschicht und damit eine wirksame Absorption der Körperflüssigkeiten zu ermöglichen.

Versuche, diese Probleme durch die Herstellung einfacher Verbundmaterialien, wie sie im Stand der Technik beschrieben sind, zu lösen, scheiterten. Gleich, ob man eine Schaumschicht auf einen Träger aufbringt, eine Schaumschicht zwischen zwei Träger einbringt oder einen Verbund, bei dem ein Trägermaterial in den Schaum eingebettet ist, vorsieht, man beobachtet stets einen Zerfall der Verbundmaterialien während des Quellungsvorgangs. Zum Teil delaminieren die Verbundmaterialien an der Grenzfläche Träger/Polymerschicht, häufiger beobachtet man jedoch, daß sich innerhalb der teilgequollenen Polymerschichten Risse bilden und sich so großflächigePolymerteile aus dem Verbundmaterial ablösen. Nach Auflösung des Verbundes quellen die abgelösten Polymerteilchen ungehindert isotrop in allen drei Raumrichtungen auf. Der vorliegenden Erfindung liegt daher die Aufgabe zugrunde, ein Absorberelement auf Basis eines Verbundmaterials aus einem Träger und einem wasserabsorbierenden Polymerschaum zur Verfügung zu stellen, das in der Lage ist, Flüssigkeiten effektiv und rasch aufzunehmen, ohne daß sich der Verbund während des Quellvorgangs auflöst.

Überraschenderweise wurde nun gefunden, daß diese Aufgabe gelöst wird durch ein Absorberelement, das sich im wesentlichen nur eindimensional ausdehnt und zwar senkrecht zum Trägermaterial.

Gegenstand der vorliegenden Erfindung ist daher ein Absorberelement aus wenigstens einem Verbundmaterial mit absorbierenden Elementen auf einem Träger, wobei auf wenigstens einem Träger mehrere Elemente aus einem superabsorbierenden Schaum (Schaumelemente) die einen Querschnitt im Bereich von 1 bis 400 mm$^2$ aufweisen, rasterförmig in einem solchen Abstand angeordnet sind, der so groß ist wie oder um bis zu 50 % geringer oder um bis zu 100 % größer ist als der Abstand, der erforderlich ist, daß sich die Elemente in gequollenem Zustand an ihren Umfängen berühren, das erhältlich ist durch

(I) Schäumen einer polymerisierbaren wäßrigen Mischung, die enthält:

(a) Säuregruppen enthaltende monoethylenisch ungesättigte Monomere, die zu mindestens 50 Mol-% neutralisiert sind,
(b) Vernetzer,
(c) wenigstens einen Initiator, und
(d) 0,1 bis 20 Gew.-% mindestens eines Tensids,

wobei das Schäumen durch Dispergieren von feinen Blasen eines gegenüber Radikalen inerten Gases erfolgt, und

(II) Aufbringen der geschäumten Mischung in dem gewünschten Rastermuster auf den Träger, und

(III) Polymerisieren der geschäumten Mischung unter Bildung eines schaumförmigen Hydrogels und gegebenenfalls Einstellen des Wassergehalts des schaumförmigen Polymerisats auf 1 bis 60 Gew.-%.

Das Rastermuster ist frei wählbar. Es muß jedoch dafür Sorge getragen werden, daß die einzelnen Schaumelemente so voneinander beabstandet sind, daß ausreichend Raum zur Verfügung steht, um ein vollständiges Ausquellen der Schaumelemente zu ermöglichen. Der Abstand zwischen den Schaumelementen wird vorzugsweise so gewählt, daß sie nach beendetem Aufquellvorgang miteinander in Berührung stehen. Ist der isotrope Quellungsfaktor beispielsweise 3,0, so muß - bei Schaumelementen in Form von Noppen (Zylindern) - der Abstand zwischen den Schaumelementen dem doppelten Durchmesser der Noppen entsprechen. Es muß also um das ursprüngliche Schaumelement herum ringförmig die 8-fache freie Fläche des ursprünglichen Schaumelementes zur Verfügung stehen.
Es kann aber auch von Vorteil sein, die Abstände zwischen den Schaumelementen um bis zu 50%, insbesondere bis zu 20% geringer zu wählen als der Abstand, der erforderlich ist, damit sich die Schaumelemente in gequollenem Zustand gerade berühren. Auf diese Weise kann eine zusätzliche Stabilität des ausgequollenen Verbundmaterials erzielt werden.
Andererseits kann es aber auch von Vorteil sein, die Abstände bis zu 100%, vorzugsweise bis zu 50%, größer zu wählen als der Abstand, der erforderlich ist, damit die Schaumelemente in gequollenem Zustand miteinander in Berührung kommen. Auf diese Weise erhält man Verbundmaterialien, die auch in ausgequollenem Zustand ein hohes Maß an Durchlässigkeit für auftreffende Flüssigkeiten aufweisen.
Durch das Aufbringen der Polymerschäume in Form eines Rasters in der erwähnten Art dehnt sich das Verbundmaterial im wesentlichen anisotrop und zwar nur in Richtung senkrecht zum Trägermaterial signifikant aus. Eine Ausdehnung des Verbundmaterials in Richtung parallel zur Oberfläche des Trägermaterials erfolgt dagegen im wesentlichen nicht.
Die Form der Schaumelemente ist im allgemeinen frei wählbar. Sie stellen im allgemeinen Erhebungen auf dem Träger dar, die zylindrisch, kalottenförmig, pyramidenförmig, kegelförmig, pyramidenstumpfförmig, kegelstumpfförmig etc sein können. Wünscht man eine erhöhte Durchlässigkeit des Verbundmaterials, dann wählt man die Form der Schaumelemente so, daß zwischen den gequollenen Schaumelementen Lücken (Zwickelvolumina) verbleiben, welche eine hohe vertikale Durchlässigkeit sicherstellen. Dies wird vorzugsweise durch die Verwendung von Schaumelementen in Form von Noppen oder noppenartigen Erhebungen oder Erhebungen mit fünfeckigem Querschnitt erreicht.
Wünscht man dagegen ein Verbundmaterial, bei dem die gequollenen Schaumelemente eine im wesentlichen geschlossene Oberfläche bilden, d.h. die Schaumelemente sind flächendeckend, so verwendet man als Schaumelemente vorzugsweise Erhebungen mit dreieckigem, quadratischem, rechteckigem oder sechseckigem Querschnitt.
Bevorzugt sind Schaumelemente mit sechseckigem oder kreisförmigem Querschnitt.
Der Flankenwinkel der Schaumelemente, d.h. der Winkel zwischen der Trägeroberfläche und der Seitenfläche des Schaumelementes, ist beliebig wählbar. Zweckmäßigerweise beträgt er 90°. In manchen Fällen ist es aber von Vorteil, wenn der Winkel kleiner ist, vorzugsweise im Bereich von 20 - 90° und insbesondere im Bereich von 50 - 90°. Es liegen dann Schaumelemente in Form eines Kegels oder einer Pyramide oder in Form eines Kegelstumpfes oder eines Pyramidenstumpfes vor.
Die Höhe der Schaumelemente richtet sich nach dem Anwendungszweck des Absorberelementes. Für Hygiene- und Sanitärartikel liegt sie im allgemeinen im Bereich von etwa 0,1 mm bis etwa 20 mm. Die Querschnittsfläche der Schaumelemente liegt im allgemeinen im Bereich von 1 bis 400 mm$^2$, vorzugsweise 2 bis 100 mm$^2$. Das Verhältnis von Höhe (in mm) zu Querschnittsfläche der Schaumelemente (in mm$^2$) liegt vorzugsweise im Bereich von 10 bis 0,01, insbesondere im Bereich von 5 bis 0,01.

Das erfindungsgemäße Absorberelement kann ein oder mehrere Verbundmaterialien umfassen. Wenn es mehrere Verbundmaterialien umfaßt, können diese in Sandwich-Art aufgebaut sein, bei denen zwischen zwei identischen oder unterschiedlichen Trägermaterialien eine strukturierte Schaumschicht eingelagert ist. Es sind jedoch auch mehrlagige Sandwich-Verbundmaterialien möglich.

Gemäß einer bevorzugten Ausführungsform umfaßt das erfindungsgemäße Absorberelement zwei Verbundmaterialien, die einander mit den die Schaumelemente tragenden Seiten so gegenüberliegen, daß die Schaumelemente des einen Verbundmaterials in die Zwischenräume zwischen den Schaumelementen des anderen Verbundmaterials hineinragen. In der Regel ist es ausreichend, die beiden Verbundmaterialien in der geschilderten Weise ineinanderzufügen. Unter Umständen kann es zur Verbesserung der Stabilität auch nützlich sein, die beiden Verbundmaterialien mit einem wasserlöslichen Kleber aneinander zu fixieren. Ein derartiges Absorberelement hat den Vorteil, daß die Aufnahmekapazität pro Volumen erheblich erhöht ist. Dabei ist es besonders zweckmäßig, Schaumelemente zu verwenden, bei denen der Flankenwinkel <90° beträgt, wie oben näher definiert.

Weiter ist es bei dieser Ausführungsform auch möglich, daß ein Verbundmaterial Schaumelemente aufweist, die in gequollenem Zustand eine geschlossene Oberfläche aufweisen, während das andere Verbundmaterial Schaumelemente aufweist, zwischen denen im gequollenen Zustand Lücken verbleiben. Dies ermöglicht einen effektiven Transport von Flüssigkeiten vertikal durch das zweite Verbundmaterial.

Als Trägermaterialien sind im Prinzip alle Materialien geeignet, die bei der Herstellung von Hygiene- oder Sanitärartikeln brauchbar sind. Sie können flüssigkeitspermeabel oder impermeabel sein. Bevorzugt sind die folgenden Materialien:

a) Folien aus Polymeren oder Metallen,
insbesondere back sheet Materialien wie Polyethylen, Polypropylen;
b) Nonwovens aus synthetischen und/oder halbsynthetischen und/oder natürlichen Fasern mit einem Flächengewicht von 5 - 400 g/m$^2$, die mit bekannten Herstellmethoden zugänglich sind.
Dazu zählen z.B. spunbonded, thermobonded, meltblown, airlaid, through airbonded und needlestitched Fasern. Eine ausführliche Zusammenstellung der Herstellung von Nonwovens findet sich in Nonwovens Industry, 1992, 37-41 und 1996, 94-95.

Auch Kombinationen aus verschiedenen Materialien, z.B. Polyester und Polyolefine, oder aus verschiedenen Fasertypen eines Materials können Verwendung finden. Zudem sind auch Composites aus Nonwovens geeignet, die mit unterschiedlichen oben erwähnten Verfahren hergestellt sind.

Die eingesetzten Nonwovens können durch geeignete Methoden permanent oder semipermanent hydrophiliert sein,

c) Papiere, wie sie z.B. als Taschentücher oder Handtücher Verwendung finden;
d) Gewebe aus synthetischen oder nichtsynthetischen Fasern
e) andere Schäume

Hier sind vor allem offenzellige Schäume von Interesse, die hydrophil sind oder hydrophiliert werden können und darüber hinaus noch weich und flexibel sind.

Als superabsorbierende Schäume sind alle bekannten, hydrophilen Schäume geeignet, die mindestens eine Aufnahmekapazität von 10 ml synthetischem Urin pro Gramm trockener Schaum und eine Absorptionsgeschwindigkeit von mindestens 0,1 g synthetischem Urin pro Gramm Schaum und Sekunde aufweisen.

Vorliegend verwendet man einen wasserabsorbierenden, vernetzten Polymerschaum, der erhältlich ist durch

(I) Schäumen einer polymersierbaren wäßrigen Mischung, die

(a) Säuregruppen enthaltende monoethylenisch ungesättigte Monomere, die zu mindestens 50 Mol-% neutralisiert sind, (a1) gegebenenfalls andere monoethylenisch ungesättigte Monomere,
(b) Vernetzer,
(c) Initiatoren.
(d) 0,1 bis 20 Gew.-% mindestens eines Tensids,
(e) gegebenenfalls mindestens einen Lösevermittler und
(f) gegebenenfalls Verdicker, Schaumstabilisatoren, Polymerisationsregler, Füllstoffe und/oder Zellkeimbilder,

wobei das Schäumen durch Dispergieren von feinen Blasen eines gegenüber Radikalen inerten Gases erfolgt, und

(II) Polymerisieren der geschäumten Mischung unter Bildung eines schaumförmigen Hydrogels und Einstellen des Wassergehalts des schaumförmigen Polymerisats auf 1 bis 60 Gew.-%.

Es wird eine polymerisierbare wäßrige Mischung zu einem Schaum verarbeitet, der verarbeitungsstabil ist und beliebig geformt werden kann. Die polymerisierbare wäßrige Mischung enthält als Komponenten (a) Säuregruppen enthaltende monoethylenisch ungesättigte Monomere, die zu mindestens 50 Mol-% neutralisiert sind. Solche Monomere sind beispielsweise monoethylenisch ungesättigte $C_3$- bis $C_{25}$-Carbonsäuren oder Anhydride, beispielsweise Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure.

Als Monomere der Gruppe (a) kommen außerdem monoethylenisch ungesättigte Sulfonsäuren in Betracht, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Vinylphosphorsäure, Allylphosphonsäure und 2-Acrylamido-2-methylpropansulfonsäure. Die Monomeren können allein oder in Mischung untereinander bei der Herstellung der superabsorbierenden Schäume eingesetzt werden. Bevorzugt eingesetzte Monomere der Gruppe (a) sind Acrylsäure, Methacrylsäure, Vinylsulfonsäure, Acrylamidopropansulfonsäure oder Mischungen dieser Säuren, z.B. Mischungen aus Acrylsäure und Methacrylsäure, Mischungen aus Acrylsäure und Acrylamidopropansulfonsäure oder Mischungen aus Acrylsäure und Vinylsulfonsäure.

Die Monomeren sind zu mindestens zu 50 Mol-% neutralisiert. Zur Neutralisation verwendet man beispielsweise Alkalimetallbasen oder Ammoniak bzw. Amine. Zur Neutralisation setzt man vorzugsweise Natronlauge oder Kalilauge ein. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydroygencarbonate oder Ammoniak vorgenommen werden. Die Säuregruppen der Monomeren werden vorzugsweise zu mindestens 65 Mol-% neutralisiert.

Die polymerisierbare wäßrige Mischung kann gegebenenfalls Monomere der Gruppe (a1) enthalten. Hierunter sollen andere monoethylenisch ungesättigte Monomere verstanden werden, die mit den Monomeren (a) und (b) copolymerisierbar sind. Hierzu gehören beispielsweise $C_2$-$C_{25}$-Olefine, die Amide und Nitrile von monoethylenisch ungesättigten Carbonsäuren, z.B. Acrylamid, Methacrylamid und N-Vinylformamid, Acrylnitril und Methacrylnitril, Dialkyldiallylammoniumhalogenide wie Dimethyldiallylammoniumchlorid, Diethyldiallylammoniumchlorid, Allylpiperidiniumbromid, N-Vinylimidazole wie z.B. N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation eingesetzt werden können. Außerdem eignen sich Dialkylaminoalkylacrylate und Dialkylaminoalkylmethacrylate, Dimethylaminoethylacrylat, Dimethylaminoethylmethacrylat, Diethylaminoethylacrylat und Diethylaminoethylmethacrylat. Die basischen Estern werden vorzugsweise in quaternisierter Form oder als Salz eingesetzt. Weitere geeignete Verbindungen der Gruppe (a1) sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z.B. Ethylvinylether oder Butylvinylether, Ester von monoethylenisch ungesättigten $C_3$- bis $C_6$-Carbonsäuren, z.B. Ester aus einwertigen $C_1$- bis $C_{25}$-Alkoholen und Acrylsäure, Methacrylsäure oder Maleinsäure, Halbester von Maleinsäure, z.B. Maleinsäuremonomethylester und Hydroxyalkylester der genannten monoethylenisch ungesättigten Carbonsäuren, z.B. 2-Hydroxyethylacrylat, Hydroxypropylacrylat, Hydroxybutylacrylat, Hydroxyethylmethacrylat, Hydroxypropylmethacrylat und Hydroxybutylmethacrylat, N-Vinyllactame wie N-Vinylpyrrolidon oder N-Vinylcaprolactam, Acrylsäure- und Methacrylsäureester von alkoxylierten einwertigen, gesättigten Alkoholen, z.B. von Alkoholen mit 10 bis 25 C-Atomen, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_N$) der Polyalkylenglykole beispielsweise bis zu 2000 betragen können. Weiterhin geeignete Monomere der Gruppe (a1) sind alkylsubstituierte Styrole wie Ethylstyrol oder tert. Butylstyrol. Die Monomeren der Gruppe (a1) können auch in Mischung bei der Copolymerisation mit den anderen Monomeren eingesetzt werden, z.B. Mischungen aus Vinylacetat und 2-Hydroxyethylacrylat in beliebigem Verhältnis.

Die Monomeren der Gruppe (b) haben mindestens 2 ethylenisch ungesättigte Doppelbindungen. Beispiele für solche Monomere, die bei Polymerisationsreaktionen üblicherweise als Vernetzer eingesetzt werden, sind N,N'-Methylen-bisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Propylenglykoldiacrylat, Butandioldiacrylat, Hexandioldiacrylat, Hexandioldimethacrylat, Diacrylate und Dimethacrylate von Blockcopolymerisaten aus Ethylenoxid und Propylenoxid, zweifach bzw. dreifach mit Acrylsäure oder Methacrylsäure veresterte mehrwertige Alkohole, wie Glycerin oder Pentaerythrit, Triallylamin, Tetrallylethylendiamin, Divinylbenzol, Diallylphthalat, Polyethylenglykoldivinylether von Polyethylenglykolen eines Molekulargewichts von 126 bis 4000, Trimethylolpropandiallylether, Butandioldivinylether, Pentaerythritttriallylether und/oder Divinylethylenharnstoff. Vorzugsweise setzt man wasserlösliche Vernetzer ein, z.B. N,N'-Methylen-bisacrylamid, Polyethylenglykoldiacrylate und Polyethylenglykoldimethacrylate, die sich von Additionsprodukten von 2 bis 400 Mol Ethylenoxid an 1 Mol eines Diols oder Polyols ableiten, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an ein Mol Glycerin, Pentaerythrittriallylether und/oder Divinylharnstoff.

Als Vernetzer kommen außerdem Verbindungen in Betracht, die mindestens eine polymerisierbare ethylenisch ungesättigte Gruppe und mindestens eine weitere funktionelle Gruppe enthalten. Die funktionelle Gruppe dieser Vernetzer muß in der Lage sein, mit den funktionellen Gruppen, im wesentlichen den Carboxylgruppen oder Sulfonsäuregruppen der Monomeren (a) zu reagieren. Geeignete funktionelle Gruppen sind beispielsweise Hydroxyl-, Amino-, Epoxi- und Aziridinogruppen.

Als Vernetzer kommen außerdem solche Verbindungen in Betracht, die mindestens zwei funktionelle Gruppen tragen, die mit Carboxyl- und Sulfonsäuregruppen der eingesetzten Monomeren der Gruppe (a) reagieren können. Die geeigneten funktionellen Gruppen wurden bereits oben genannt, d.h. Hydroxyl-, Amino-, Epoxi-, Isocyanat-, Ester-, Amide- und Aziridinogruppen. Beispiele für solche Vernetzer sind Ethylenglykol, Diethylenglykol, Triethylenglykol, Tetraethylenglykol, Polyethylenglykol, Glycerin, Polyglycerin, Propylenglykol, Diethanolamin, Triethanolamin, Polypropylenglykol, Blockcopolymerisate aus Ethylenoxid und Propylenoxid, Sorbitanfettsäureester, ethoxylierte Sorbitanfettsäureester, Trimethylolpropan, Pentaerythrit, Polyvinylalkohol, Sorbit, Polyglycidylether wie Ethylenglykoldiglycidylether, Polyethylenglykoldiglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl) propionat], 1,6-Hexamethylendiethylenharnstoff, Diphenylmethan-bis-4,4'-N,N'-diethylenharnstoff, Halogenepoxyverbindungen wie Epichlorhydrin und $\alpha$-Methylfluorhydrin, Polyisocyanate wie 2,4-Toluylendiisocyanat und Hexamethylendiisocyanat, Alkylencarbonate wie 1,3-Dioxolan-2-on und 4- Methyl-1,3-dioxolan-2-on, polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Weitere geeignete Vernetzer sind polyvalente Metallionen, die in der Lage sind, ionische Vernetzungen auszubilden. Beispiele für solche Vernetzer sind Magnesium-, Calcium-, Barium- und Aluminiumionen. Diese Vernetzer werden beispielsweise als Hydroxide, Carbonate oder Hydrogencarbonate der wäßrigen polymerisierbaren Lösung zugesetzt.

Weitere geeignete Vernetzer sind multifunktionelle Basen, die ebenfalls in der Lage sind, ionische Vernetzungen auszubilden, beispielsweise Polyamine oder deren quaternierte Salze. Beispiele für Polyamine sind Ethylendiamin, Diethylentriamin, Triethylentetramin, Tetraethylenpentamin, Pentaethylenhexamin und Polyethylenimine sowie Polyvinylamine mit Molmassen von jeweils bis zu 4000000.

In einer bevorzugten Ausführungsform der Erfindung werden zwei unterschiedliche Vernetzer eingesetzt, von denen der eine wasserlöslich und der andere wasserunlöslich ist. Der hydrophile Vernetzer, der in der wäßrigen Phase der Reaktionsmischung löslich ist, bewirkt in konventioneller Weise eine relativ gleichmäßige Vernetzung des entstehenden Polymeren, wie es bei der Herstellung eines Superabsorbers üblich ist. Der hydrophobe Vernetzer, der in der polymerisierbaren wäßrigen Mischung unlöslich bzw. darin nur begrenzt löslich ist, reichert sich in der Tensidgrenzschicht zwischen der Gasphase und der polymerisierbaren wäßrigen Phase an. Dadurch wird bei der nachfolgenden Polymerisation die Oberfläche des Schaums stärker vernetzt als der innere Teil des Superabsorberhydrogels. Man erhält dadurch einen Kern-Schale-Aufbau des Schaums unmittelbar bei der Herstellung des Superabsorberschaums. Eine solche starke oberflächliche Vernetzung eines Superabsorberschaums ist bei den bekannten Herstellverfahren des Standes der Technik nur dadurch möglich, daß man einen bereits gebildeten schaumförmigen Superabsorber nachträglich oberflächlich vernetzt. Für diese Nachvernetzung ist bei konventioneller Arbeitsweise ein eigener Prozeßschritt notwendig, der bei dem Verfahren der vorliegenden Erfindung entfallen kann.

Erfindungsgemäße Produkte mit einem Kern-Schale-Aufbau zeigen gegenüber homogen vernetzten Proben hinsichtlich der Aufnahmegeschwindigkeit, Verteilungswirkung und Gelstabilität deutlich verbesserte Eigenschaften. Mit Ausnahme polyvalenter Metallionen sind alle oben beschriebenen wasserunlöslichen Vernetzer, die den unterschiedlichen Gruppen zugeordnet werden können, geeignet, um Schäume mit einem Kern-Schale-Aufbau herzustellen, d.h. Schäume, bei denen die gesamte Oberfläche stärker vernetzt ist als die darunter liegende Schicht, die oben als Kernschicht bezeichnet wurde. Besonders bevorzugte hydrophobe Vernetzer sind Diacrylate oder Dimethacrylate oder Divinylether von Alkandiolen mit 2 bis 25 C-Atomen (verzweigt, linear, mit beliebiger Anordnung der OH-Gruppen) wie z.B. 1,3-Propandiol, 1,4-Butandiol, 1,6-Hexandiol, Neopentylglycol, 1,9-Nonandiol oder 1,2-Dodecandiol, Di-, Trioder Polypropylenglycoldiacrlyate oder -dimethacrylate, Allylacrylat, Allylmethacrylat, Divinylbenzol, Glycidylacrylat oder Glycidylmethacrylat, Allylglycidylether und Bisglycidylether der oben aufgeführten Alkandiole.

Geeignete hydrophile Vernetzer sind beispielsweise N,N'-Methylenbisacrylamid, Polyethylenglycoldiacrylate oder -dimethacrylate mit einem Molekulargewicht $M_N$ von 200 bis 4000, Dinvinylharnstoff, Triallylamin, Diacrylate oder Dimethacrylate von Additionsprodukten von 2 bis 400 Mol Ethylenoxid an 1 Mol eines Diols oder Polyols oder das Triacrylat eines Additionsprodukts von 20 Mol Ethylenoxid an 1 Mol Glycerin und Vinylether von Additionsprodukten von 2 bis 400 Mol Ethylenoxid an 1 Mol eines Diols oder Polyols.

Die Monomeren der Gruppe (a) sind in der polymerisierbaren wäßrigen Mischung beispielsweise in Mengen von 10 bis 80 und vorzugsweise 20 bis 60 Gew.-% enthalten. Die Monomeren der Gruppe (a1) werden nur gegebenenfalls zur Modifizierung der Superabsorberschäume eingesetzt und können in Mengen bis zu 50, vorzugsweise in Mengen bis zu 20 Gew.-% in der polymerisierbaren wäßrigen Mischung enthalten sein. Die Vernetzer (b) sind in der Reaktionsmischung beispielsweise von 0,001 bis 5 und vorzugsweise von 0,01 und 2 Gew.-% vorhanden.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wäßrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden.

Als Polymerisationsinitiatoren können sämtliche unter den Polymerisationsbedingungen in Radikale zerfallende Verbindungen eingesetzt werden, z.B. Peroxide, Hydroperoxide, Wasserstoffperoxid, Persulfate, Azoverbindungen und die sogenannten Redoxkatalysatoren. Bevorzugt ist der Einsatz von wasserlöslichen Initiatoren. In manchen Fällen ist es vorteilhaft, Mischungen verschiedener Polymerisationsinitiatoren zu verwenden, z.B. Mischungen aus Wasserstoffperoxid und Natrium- oder Kaliumperoxidisulfat. Mischungen aus Wasserstoffperoxid und Natriumperoxidisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, Di-(2-ethylhexyl)-peroxidicarbonat, Dicyclohexylperoxydicarbonat, Di-(4-tert.-butylcyclohexyl)peroxidicarbonat, Dimyristil-peroxidicarbonat, Diacetylperoxydicarbonat, Allylperester, Cumylperoxyneodecanoat, tert.-Butylper-3,5,5-tri-methylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z.B. 2,2'-Azobis-(2-amidinopropan)dihydrochlorid, 2,2'-Azobis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z.B. in Mengen von 0,01 bis 5, vorzugsweise 0,1 bis 2,0 Gew.-%, bezogen auf die zu polymerisierenden Monomeren.

Als Initiatoren kommen außerdem Redoxkatalysatoren in Betracht. Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetall-hydrogensulfit, -sulfit, -thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise $3 \cdot 10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte $\alpha$-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-2-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Die polymerisierbaren wäßrigen Mischungen enthalten als Komponente (d) 0,1 bis 20 Gew.-% mindestens eines Tensids. Die Tenside sind für die Herstellung und die Stabilisierung des Schaums von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additionsprodukte von 80 Mol Ethylenoxid an 1 Mol Talgfettalkohol. Weitere handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere handelsübliche nichtionische Tenside werden durch Ethoxylierung von Rizinusöl erhalten. Pro Mol Rizinusöl werden beispielsweise 12 bis 80 Mol Ethylenoxid angelagert. Weitere handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$-Oxoalkohols, oder die Umsetzungsprodukte von 7 bis 8 Mol Ethylenoxid an 1 Mol eines $C_{13}/C_{15}$-Oxoalkohols. Wietere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines $C_{12}$- bis $C_{18}$-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten $C_{13}/C_{15}$-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche anionische Tenside. Weitere geeignete anionische Tenside sind Schwefelsäurehalbester von $C_{13}/C_{15}$-Oxoalkoholen, Paraffinsulfonsäuren wie $C_{15}$-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure sowie Fettalkoholphosphate wie $C_{15}/C_{18}$-Fettalkoholphosphat. Die polymerisierbare wäßrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt der polymerisierbaren wäßrigen Mischung beträgt 0,1 bis 20, vorzugsweise 0,5 bis 10 Gew.-%. In den meisten Fällen weisen die polymerisierbaren wäßrigen Mischungen einen Tensidgehalt von 1,5 bis 6 Gew.-% auf.

Die polymerisierbaren wäßrigen Mischungen können als Komponente (e) gegebenenfalls mindestens einen Lösevermittler enthalten. Hierunter sollen mit Wasser mischbare organische Lösemittel verstanden werden, z.B. Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glycerinmonomethylether.

Die polymerisierbaren wäßrigen Mischungen enthalten 0 bis 50 Gew.-% mindestens eines Lösevermittlers. Falls Lösevermittler eingesetzt werden, beträgt ihr Gehalt in der polymerisierbaren wäßrigen Mischung vorzugsweise bis 25 Gew.-%.

Die polymerisierbare wäßrige Mischung kann gegebenenfalls Verdicker, Schaumstabilisatoren, Polymerisationsregler, Füllstoffe und Zellkeimbildner enthalten. Verdicker werden beispielsweise zur Optimierung der Schaumstruktur und zur Verbesserung der Schaumstabilität eingesetzt. Man erreicht damit, daß der Schaum während der Polymerisation nur geringfügig schrumpft. Als Verdikkungsmittel kommen alle hierfür bekannten natürlichen und synthetischen Polymeren in Betracht, die die Viskosität eines wäßrigen Systems stark erhöhen. Hierbei kann es sich um wasserquellbare oder wasserlösliche synthetische und natürliche Polymere handeln. Als Verdicker sind auch pulverförmige Superabsorber geeignet. Eine ausführliche Übersicht über Verdicker findet man beispielsweise in den Veröffentlichungen von R.Y. Lochhead und W.R. Fron, Cosmetics&Toileteris, 108, 95-135 (Mai 1993) und M.T. Clarke, "Rheological Additives" in D. Laba (ed.) "Rheological Properties of Cosmetics and Toiletris", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polymerisate der oben unter (a) beschriebenen Säuregruppen enthaltenden monoethylenisch ungesättigten Monomeren. Solche Verdicker sind beispielsweise hochmolekulare Homopolymerisate von Acrylsäure und/oder Methacrylsäure oder geringfügig vernetzte Copolymerisate aus Acrylsäure und/oder Methacrylsäure und einer Verbindung, die mindestens 2 ethylenisch ungesättigte Doppelbindungen enthält, z.B. Butandioldiacrylat. Außerdem eignen sich hochmolekulare Polymerisate von Acrylamid und Methacrylamid oder Copolymerisate aus Acrylsäure und Acrylamid mit Molmassen von mehr als 1 Million. Solche Copolymerisate sind als Verdickungsmittel bekannt. Auch hochmolekulare Polyethylenglykole oder Copolymerisate aus Ethylenglykol und Propylenglykol sowie hochmolekulare Polysaccharide wie Stärke, Guarkernmehl, Johannisbrotkernmehl oder Derivate von Naturstoffen wie Carboxymethylcellulose Hydroxyethylcellulose, Hydroxymethylcellulose, Hydroxypropylcellulose und Cellulose Mischether sind bekannte Verdicker. Eine weitere Gruppe von Verdickern sind wasserunlösliche Produkte, wie feinteiliges Siliciumdioxid, pyrogene Kieselsäuren, Fällungskieselsäuren in hydrophilen oder hydrophoben Modifikationen, Zeolithe, Titandioxid, Cellulosepulver, oder andere von Superabsorbern verschiedene feinteilige Pulver von vernetzten Polymerisaten. Die polymerisierbaren wäßrigen Mischungen können die Verdicker in Mengen bis zu 30 Gew.-% enthalten. Falls solche Verdickungsmittel überhaupt eingesetzt werden, sind sie in Mengen von 0,1, vorzugsweise 0,5 bis 20 Gew.-% in der polymerisierbaren wäßrigen Mischung enthalten.

Um die Schaumstruktur zu optimieren, kann man gegebenenfalls Kohlenwasserstoffe mit mindestens 5 C-Atomen im Molekül zu der wäßrigen Reaktionsmischung zusetzen. Geeignete Kohlenwasserstoffe sind beispielsweise Pentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wäßrigen Mischung während des Schäumens liegt. Die aliphatischen Kohlenwasserstoffe erhöhen die Standzeit der noch nicht polymerisierten geschäumten wäßrigen Reaktionsmischung. Dadurch wird das Handling der noch nicht polymerisierten Schäume erleichtert und die Prozeßsicherheit erhöht. Die Kohlenwasserstoffe werden in Mengen von 0 bis 10 Gew.-%, bezogen auf die polymerisierbare wäßrige Mischung eingesetzt. Im Fall ihres Einsatzes betragen die bevorzugt in der wäßrigen Mischung vorliegenden Mengen 0,1 bis 5 Gew.-%.

Um die Eigenschaften der Superabsorber zu variieren, beispielsweise die Aufnahmegeschwindigkeit und die Aufnahmekapazität von Wasser, kann es von Vorteil sein, der wäßrigen Reaktionsmischung einen Polymerisationsregler oder eine Mischung mehrerer Polymerisationsregler zuzusetzen. Geeignete Polymerisationsregler sind beispielsweise Ameisensäure, Thioverbindungen wie 2-Mercaptoethanol, Mercaptopropanol, Mercaptobutanol, Dodecylmercaptan, Thioglykolsäure oder Amine wie Ethanolamin, Diethanolamin, Triethanolamin, Triethylamin, Morpholin oder Piperidin. Die Mengen an Polymerisationsregler können bis zu 10 Gew.-%, bezogen auf die eingesetzten Monomeren, betragen. Falls Polymerisationsregler eingesetzt werden, verwendet man vorzugsweise 0,1 bis 5 Gew.-%, bezogen auf die Monomeren.

Die unter (f) angegebenen fakultativ zu verwendenden Bestandteile können einzeln, oder in Mischung bei der Herstellung der erfindungsgemäßen Polymerisate eingesetzt werden. Man kann jedoch auch in Abwesenheit von Verdickern, Schaumstabilisatoren, Füllstoffe, Zellkeimbildner und Polymerisationsreglern arbeiten.

Bei der erfindungsgemäßen Herstellung von wasserabsorbierenden, schaumförmigen, vernetzten Polymerisaten wird in einer ersten Verfahrensstufe die oben beschriebene polymerisierbare wäßrige Mischung geschäumt. Zu diesem Zweck wird in der wäßrigen Monomerphase ein gegenüber Radikalen inertes Gas in Form von feinen Blasen in der Art dispergiert, daß sich ein Schaum bildet. Das Eintragen von Gasblasen in die Monomermischung gelingt beispielsweise mit Hilfe von Schlag-, Schüttel-, Rühr- oder Peitschvorrichtungen. Ferner ist es möglich solche Schäume dadurch herzustellen, daß Gase aus einer flüssigkeitsbedeckten öffnung ausströmen oder durch das Ausnutzen von Turbulenzerscheinungen in Strömungen. Schließlich kann auch die Ausbildung von Lamellen an Drähten oder Sieben für diesen Zweck genutzt werden. Diese unterschiedlichen Methoden können auch gegebenenfalls miteinander kombiniert werden. Als gegenüber Radikalen inerte Gase eignen sich beispielsweise Stickstoff, Kohlendioxid, Helium, Neon und Argon. Vorzugsweise verwendet man Stickstoff.

Die Herstellung des Schaums erfolgt erfindungsgemäß getrennt von der Polymerisation. Die polymerisierbare wäßrige Mischung kann beispielsweise in technischen Apparaturen geschäumt werden, die für die Herstellung von Harnstoff-Formaldehyd-Schäumen bekannt sind, vgl. Frisch und Saunders, Polymeric Foams Part II, S. 679 ff (1973). Das Schäumen der polymerisierbaren wäßrigen Mischung kann im Labor im einfachsten Fall in einer konventionellen Küchenmaschine erfolgen, die mit Schneebesen bestückt ist. Die Schlagschaumerzeugung wird vorzugsweise in einer Inertgasatmosphäre durchgeführt. Als Inertgase sind beispielsweise Stickstoff, Edelgase oder Kohlendioxid verwendbar. Zur Herstellung des Schaums werden alle Komponenten der Reaktionsmischung vereinigt. Zweckmäßigerweise geht man dabei so vor, daß zunächst alle wasserlöslichen Komponenten in Wasser gelöst werden und daß man erst danach die wasserunlöslichen Stoffe zusetzt. Je nach verwendetem Verfahren der Schlagschaumerzeugung und in Abhängigkeit von dem in der polymerisierbaren wäßrigen Mischung enthaltenen Initiator kann es auch von Vorteil sein, den Initiator erst am Ende des Aufschlagprozesses der Mischung zuzusetzen. Die Konsistenz der Schlagschäume kann in einem weiteren Bereich variiert werden. So ist es möglich, leichte fließfähige Schlagschäume oder aber steife, schnittfeste Schäume herzustellen. Ebenso kann man die mittlere Größe der Gasblasen, ihre Größenverteilung und ihre Anordnung in der Flüssigkeitsmatrix durch die Auswahl der Tenside, der Lösevermittler, Verdicker und Schaumstabilisatoren, Zellkeimbildner, der Temperatur und der Aufschlagtechnik in einem weiten Bereich variieren, so daß man in einfacher Weise Dichte, Offenzelligkeit oder Wandstärke des Matrixmaterials einstellen kann. Die Temperaturen der polymerisierbaren wäßrigen Mischung liegen während des Schäumvorgangs in dem Bereich von -10 bis 100, vorzugsweise 0 bis +50°C. In jedem Falle werden bei der Schaumerzeugung Temperaturen angewandt, die unterhalb des Siedepunkts von Bestandteilen der polymerisierbaren wäßrigen Mischung liegen. Die Schaumerzeugung kann auch unter erhöhtem Druck erfolgen, z.B. bei 1,5 bis 25 bar. Bevorzugt wird jedoch unter Atmosphärendruck gearbeitet.

Der besonders bevorzugte Schaum ist in der deutschen Patentanmeldung P 195 409 51.5 der Anmelderin beschrieben.

Die Schaumelemente werden direkt auf dem Träger erzeugt.

Die Herstellung erfolgt zweckmäßigerweise dadurch, daß der ggf. aufgeschlagene Monomerschaum auf das Trägermaterial in dem gewünschten Rastermuster und in einer Dicke von 0.05 bis 20 mm, bevorzugt 0.1 bis 10 mm aufgebracht und anschließend auspolymerisiert wird. Das Aufbringen der Schaumelemente ist mit allen gängigen Techniken möglich, z.B. kann es mit Hilfe von Schablonen, durch Drucktechniken wie z.B. Siebdruckverfahren oder auch durch gezieltes Aufspritzen einzelner Schaumelemente z.B. mit Hilfe des Ink-Jet-Verfahrens erfolgen. Die so erhaltenen, frei stehenden Schaumelemente können anschließend in geeigneter Weise auspolymerisiert werden, wie es in den oben genannten Patenten und Patentanmeldungen beschrieben ist. Die Polymerisation der bevorzugten Schäume erfolgt wie in der P 195 40 951.5 beschrieben. Sie kann je nach verwendetem Initiator durch Temperaturerhöhung, durch Lichteinwirkung, durch Bestrahlen mit Elektronenstrahlen oder auch durch Temperaturerhöhung und Lichteinwirkung erfolgen. Um die Temperatur der geschäumten polymerisierbaren wäßrigen Mischung zu erhöhen, kann man alle in der Technik üblichen Verfahren anwenden, beispielsweise den Schaum mit heizbaren Platten in Kontakt bringen, Einwirkung von Infrarotbestrahlung auf den polymerisierbaren Schaum oder Beheizen mit Hilfe von Mikrowellen.

Falls dickere Schichten eines Schaumstoffs hergestellt werden sollen, z.B. Schäume mit Dicken von mehreren Zentimetern, ist die Erwärmung des polymerisierbaren geschäumten Materials mit Hilfe einer Mikrowelle besonders vorteilhaft, weil auf diesem Wege eine relativ gleichmäßige Erwärmung erreicht werden kann.

Die Polymerisation erfolgt beispielsweise bei Temperaturen von 20 bis 180, vorzugsweise in dem Bereich von 20 bis 100°C.

Bei Initiierung der Polymerisation durch Lichteinwirkung auf das geschäumte polymerisierbare Material kann man alle konventionellen Belichtersysteme anwenden, sofern ihr Emissionsspektrum an den eingesetzten Photoinitiator adaptiert ist. Bei einem Start der Polymerisation durch Belichten wird vorteilhaft eine Kombination eines Photoinitiators eines thermischen Initiators oder/und aber ein Photoinitator eingesetzt, der auch als thermischer Initiator wirken kann, z.B. Azoinitiatoren. Da sich der Schaum während der Polymerisation durch die hohe Polymerisationswärme stark erwärmt, wird auf diese Weise ein besonders schneller und effektiver Ablauf der Polymerisationsreaktion erreicht. Bei der Initiierung durch Lichteinwirkung liegt die Polymerisationstemperatur in dem Bereich von 0 bis 150, vorzugsweise 10 bis 100°C.

Ein wesentlicher Vorteil des erfindungsgemäßen Verfahrens ist darin zu sehen, daß die Polymerisation unter weitgehendem Erhalt der Struktur der geschäumten polymerisierbaren wäßrigen Mischung abläuft, d.h. der polymerisierbare Schaum verändert während der Polymerisation sein Volumen nur unwesentlich. Die Polymerisationsreaktion wird durch die Starttemperatur, die Initiierungstechnik oder die Wärmeabfuhr beeinflußt. Die Polymerisationstemperatur wird vorzugsweise dahingehend kontrolliert, daß ein Sieden der polymerisierbaren wäßrigen Mischung vermieden wird. Mit fortschreitender Polymerisation tritt eine Verfestigung des Schaums infolge zunehmender Gelbildung ein. Nach Beendigung der Polymerisation liegt ein schaumförmiges Hydrogel vor, daß einen Wassergehalt von 30 bis 80 Gew.-% hat. Der Schaum hat zumindest teilweise eine offenzellige Struktur. Für die Anwendung des Schaums als Superabsorber ist eine Restfeuchte von 1 bis 45, vorzugsweise 15 bis 35 Gew.-% wünschenswert. Das bei der Polymerisation anfallende schaumförmige Hydrogel wird daher meistens getrocknet. Um einen flexiblen Schaum zu erhalten, muß der Schaum eine gewisse Restfeuchte aufweisen. Der Wassergehalt hängt stark von der Dichte des erzeugten Schaums ab. Je höher die Dichte ist, desto mehr Restfeuchte ist einzustellen. Daher kann eine Obergrenze von 35 bis 45 Gew.-% Wasser durchaus sinnvoll sein. Wird ein Ansatz mit sehr hohem Festgehalt polymerisiert, der einen Schaum mit einer sehr hohen Dichte ergibt, kann es sogar notwendig sein, nach der Polymerisation den Schaum weiter anzufeuchten, um die notwendige Flexibilität zu erhalten.

Der Schaum kann mit Hilfe aller konventionellen Techniken getrocknet werden, beispielsweise durch Erhitzen mit einem heißen Gasstrom, durch Anlegen von Vakuum, durch Infrarotbestrahlung oder durch Erhitzen mit Mikrowellenstrahlung. Die Mikrowellenstrahlung erweist sich auch hier wiederum beim Trocknen von großvolumigen Formkörpern als vorteilhaft.

Als Alternative zu vorstehendem Vorgehen kann man bei relativ dünnflüssigen Schlagschäumen den Schaum mittels einer Schablone auftragen und die Schablone bis nach der Polymerisation auf dem Trägermaterial belassen.

Nach dem oben beschriebenen Verfahren erhält man einen überwiegend oder zumindest teilweise offenzelligen Superabsorberschaum, der in vollständig getrocknetem Zustand relativ hart und spröde ist. Für viele Anwendungen werden jedoch Schäume bzw. Schaumelemente verlangt, die flexibel sind. Dies kann mit Hilfe von externen Weichmachern oder durch eine interne Flexibilisierung geschehen.

Externe Weichmacher sind Komponenten, die zusätzlich zu den gelbildenden Komponenten entweder der Reaktionsmischung vor dem Verschäumen zugesetzt werden, oder die nachträglich auf den Schaum aufgetragen werden. Als Weichmacher kommen beispielsweise hydrophile und hygroskopische Substanzen in Betracht. Eine externe Flexibilisierung wird in erster Linie durch das gezielte Einstellen eines bestimmten Restwassergehalts erreicht. Weiterhin kann die Flexibilisierung durch den Einsatz von beispielsweise Polyolen wie Glycerin, Polyalkylenglykolen wie Polyethylenglykolen oder Polypropylenglykolen, oder kationischen Tensiden verbessert werden. Geeignete kationische Tenside sind beispielsweise mit Dimethylsulfat quaternierte Umsetzungsprodukte von 1 Mol Oleylamin mit 5 bis 10 Mol Ethylenoxid, Distearyldimethylammoniumchlorid, Lauryltrimethylammoniumchlorid, Cetylpyridiniumbromid und Ethanolaminester langkettiger Fettsäuren wie Stearinsäurediethanolaminester, Stearinsäuremonoethanolaminester und Stearinsäuretriethanolaminester, der bevorzugt als externer Weichmacher eingesetzt wird.

Unter interner Flexibilisierung des Schaums wird der Einsatz von weichmachenden Komponenten verstanden, die in die Gelstruktur eingebaut werden. Hierbei kann es sich um Substanzen handeln, die selbst ungesättigte Gruppen tragen und bei der Polymerisation als Monomere (b) in der polymerisierbaren wäßrigen Mischung vorliegen und mit in die Gelstruktur eingebaut werden oder daß sie mit dem gelbildenden Material reagieren. Der interne Weichmacher soll eine Erniedrigung der Glastemperatur des Polymeren bewirken, das den Superabsorber darstellt. Als interne Weichmacher sind beispielsweise Olefine, Ester aus ethylenisch ungesättigten $C_3$ bis $C_5$-Carbonsäuren und einwertigen $C_2$ bis $C_{30}$-Alkoholen oder Polyethylenglykol- oder Polypropylenglykolmonoester von monoethylenisch ungesättigten $C_3$- bis $C_5$-Carbonsäuren geeignet. Zur internen Flexibilisierung eignen sich diejenigen Monomeren (b), die die Glastemperatur der entstehenden Copolymerisate mit den Monomeren (a) herabsetzen, z.B. Vinylester von mindestens 4 C-Atome enthaltenden gesättigten Carbonsäuren, Alkylvinylether mit mindestens 2 C-Atomen in der Alkylgruppe, Vinyllactame und alkylsubstituierte Styrole wie Ethylstyrol.

Wie oben bereits angegeben wurde, kann eine inhomogene Vernetzungsdichte bei den erfindungsgemäßen Superabsorberschäumen bereits während der Herstellung erzeugt werden. Dies ist besonders vorteilhaft, wenn man als Monomere der oben beschriebenen Komponenten

(a) Acrylsäure, Methacrylsäure, Vinylsulfonsäure, Acrylamidopropansulfonsäure oder deren Mischungen und

(c) eine Mischung aus mindestens einem wasserlöslichen und mindestens einem wasserunlöslichen Vernetzer einsetzt.

Dennoch kann es wünschenswert sein, den Vernetzungsgrad des Schaumes nachträglich zu verändern. Um dieses Ziel zu erreichen, kann man beispielsweise während der Polymerisation durch den Zusatz geeigneter Monomere in das Gel latente Vernetzungsstellen einbauen, die unter den Bedingungen der Schaumherstellung nicht zu Vernetzungsreaktionen führen, jedoch unter speziellen Bedingungen, die nachträglich angewandt werden können, z.B. durch stark erhöhte Temperatur, in der Lage sind, weitere Vernetzungspunkte in der Gelstruktur zu bilden. Als Beispiele für solche Monomere kann der Einbau von Hydroxygruppen enthaltenden Verbindungen dienen, die bei höherer Temperatur, d.h. bei Temperaturen oberhalb von 150°C in der Lage sind, mit den Carboxylgruppen in der Schaumstruktur zu reagieren. Geeignete Verbindungen, die latente Vernetzungsstellen aufweisen, sind beispielsweise Hydroxyethylacrylat, Hydroxypropylacrylat, Hydroxybutylacrylat, Monoacrylsäureester des Glycerins, Monoacrylate oder Monomethacrylate von Polyethylenglykolen mit mindestens 2 Ethylenglykoleinheiten, Monoacrylate oder Monomethacrylate von Polypropylenglykolen mit mindestens 2 Propylenglykoleinheiten und Monomethacrylate von mehrwertigen Alkoholen, z.B. Hydroxybutylmethacrylat, Hydroxypropylmethacrylat, Hydroxyethylmethacrylat oder Glycerinmonomethacrylat.

Als weitere Möglichkeit einer homogenen Nachvernetzung bietet sich der nachträgliche Zusatz von Vernetzungsreagenzien an, d.h. Verbindungen, die mindestens zwei reaktive Gruppen aufweisen, die unter geeigneten Bedingungen in der Lage sind, z.B. beim Erhitzen auf Temperaturen oberhalb von 70°C, mit den Säuregruppen des schaumförmigen Hydrogel zu reagieren. In diesem Falle ist es auch möglich, gesteuert über die Eindringtiefe des Vernetzers, eine Modifikation der inhomogenen Vernetzungsdichte zu erreichen. Geeignete Vernetzer bilden mit den Carboxylgruppen der Polymermatrix kovalente oder ionische Bindungen. Geeignete Vernetzungsmittel sind Verbindungen, die mindestens zwei funktionelle Gruppen der gleichen oder unterschiedlicher Art aufweisen, z.B. Hydroxy-, Amino-, quaternäre Ammonium-, Isocyanato-, Epoxi-, Aziridino-, Ester- oder Amidgruppen. Bevorzugte Nachvernetzungsmittel sind Polyalkohole wie Glycerin oder Bisepoxide. Das Auftragen der Vernetzungsmittel auf das geschäumte Material kann beispielsweise durch Sprühen, Tauchen oder durch Gasphasenabscheidung erfolgen.

Die erfindungsgemäßen Absorberelemente können in allen Arten von Hygiene- oder Sanitärartikeln oder in Wundverbänden Anwendung finden, beispielsweise Babywindeln, Damenbinden oder Inkontinenzprodukten. Die Ausgestaltung derartiger Artikel erfolgt in üblicher Weise, beispielsweise wie in der WO-A-94/22502 beschrieben. Die Absorberelemente können anstelle der in dieser Publikation beschriebenen superabsorbierenden Schaumschichten eingesetzt werden.

Die Erfindung wird nachfolgend anhand der Figuren 1 bis 4 sowie der Beispiele näher erläutert, ohne darauf beschränkt zu sein.

Es zeigen:

Fig. 1    eine schematische Aufsicht auf ein Absorberelement aus einem Verbundmaterial vor und nach dem Quellen;

Fig. 2    einen schematischen Querschnitt durch das Absorberelement der Fig. 1 vor und nach dem Quellen;

Fig. 3    einen schematischen Querschnitt durch ein Absorberelement aus zwei Verbundmaterialien vor dem Quellen;

Fig. 4    einen schematischen Querschnitt durch das Absorberelement nach Fig. 3 nach dem Ausquellen.

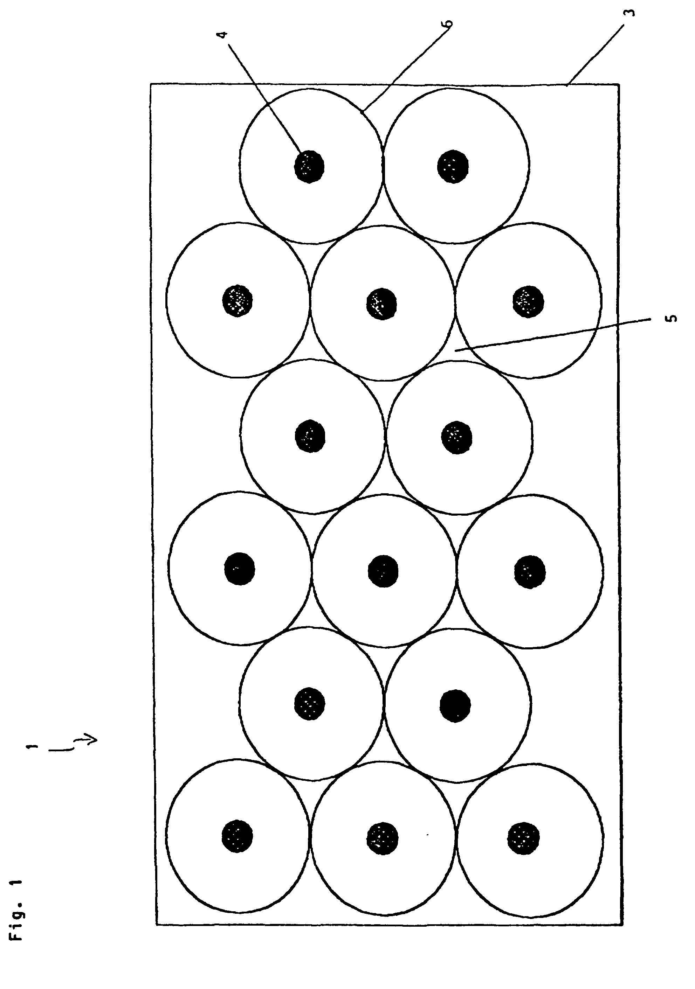

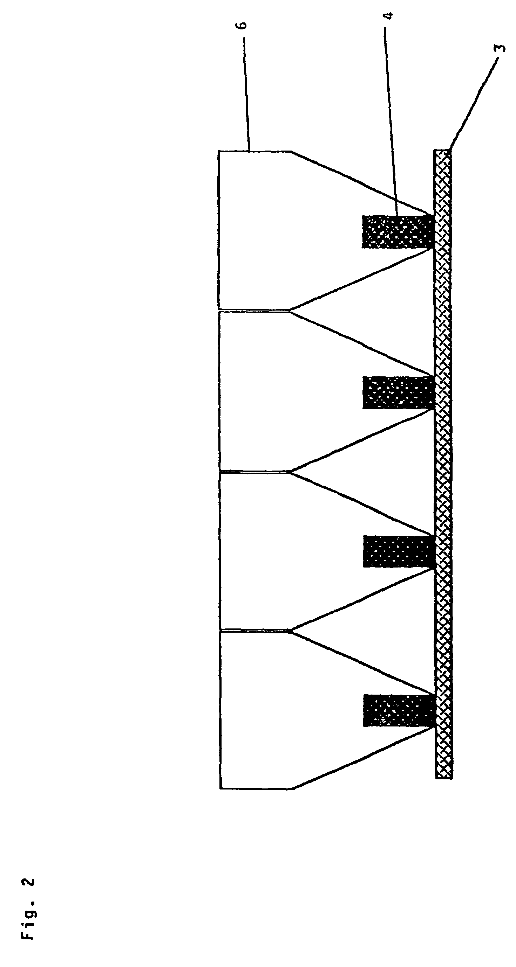

Fig. 1 zeigt ein Absorberelement aus einem Verbundmaterial 1 aus einem Trägervlies 3, auf dem sind mehrere in Form von Noppen vorliegende Schaumelemente 4 aufgebracht sind. Bei Aufnahme von Flüssigkeit quellen die Schaumelemente 4, d.h. man erhält die gequollene Schaumelemente 6 mit wesentlich größerem Querschnitt. Der Abstand der Schaumelemente 4 ist so gewählt, daß sie sich nach dem Quellen berühren, wie dies aus Fig. 1 und aus Fig. 2 ersichtlich ist. Zwischen den gequollenen Schaumelementen 6 verbleiben Lücken 5, die eine hohe vertikale Durchlässigkeit für die zu absorbierende Flüssigkeit sicherstellen. Die Figur 2 zeigt, daß sich die Schaumelemente 4 nahe der Trägeroberfläche nur begrenzt ausdehnen, während sie sich mit größer werdendem Abstand vom Träger zunehmend unbehindert ausdehnen können, so daß die gequollenen Schaumelemente 6 die in Fig. 2 gezeigte konische Struktur aufweisen. Somit kann sich das Verbundmaterial 1 bzw. das Absorberelement nur in einer Richtung ausdehnen und zwar senkrecht zur Oberfläche des Trägers 3.

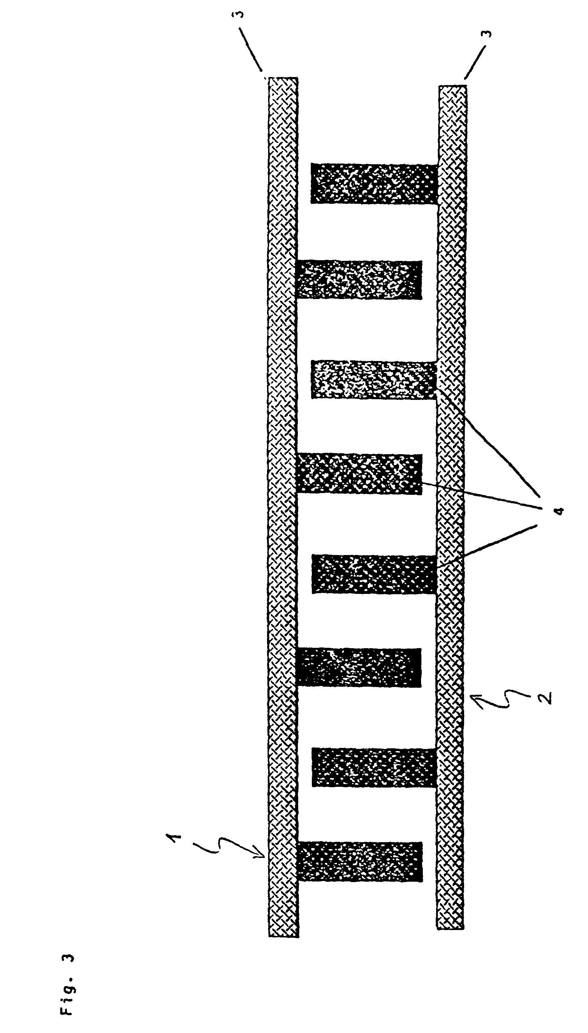

Die Figur 3 zeigt ein Absorberelement aus zwei Verbundmaterialien 1 und 2. Auf jedem Träger 3, die unterschiedlicher Natur sein können, sind Schaumelemente 4 der in den Fig. 1 und 2 gezeigten Art aufgebracht. Die beiden Verbundmaterialien werden so miteinander kombiniert, daß die Schaumelemente 4 des einen Verbundmaterials 1 in die Zwischenräume zwischen den Schaumelementen 4 des anderen Verbundmaterials 2 hineinragen. Das resultierende Absorberelement hat den Vorteil, daß die Aufnahmekapazität für Flüssigkeiten auf diese Weise etwa verdoppelt wird.

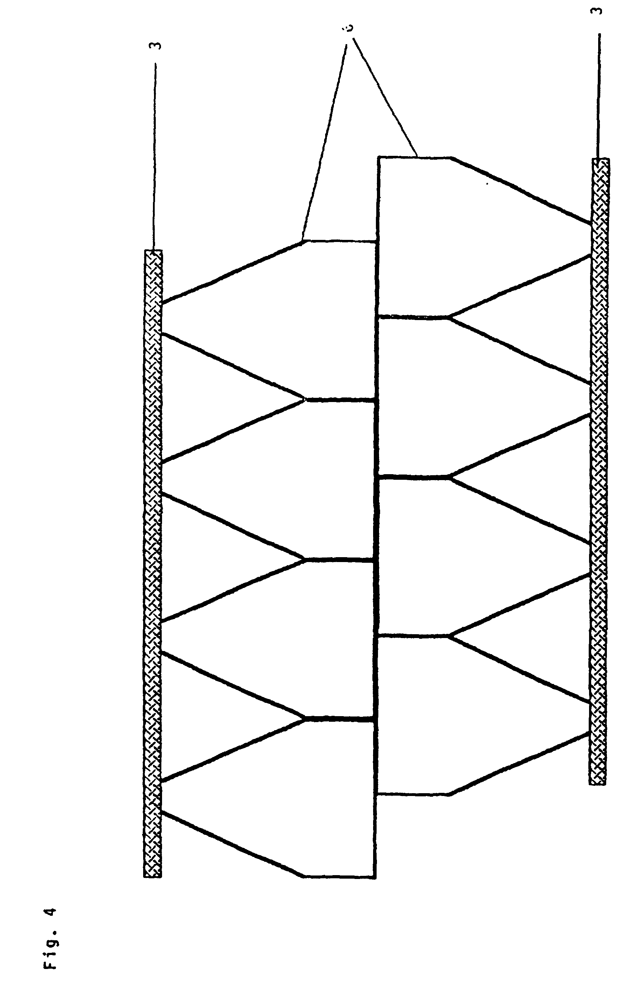

Fig. 4 zeigt das Absorberelement der Figur 3 nach dem Ausquellen. Es ist ersichtlich, daß eine Ausdehnung nur senkrecht zur Trägeroberfläche erfolgt ist, die Gestalt der gequollenen Schaumelemente 6 ist wie in Fig. 2 gezeigt. Die beiden Verbundmaterialien werden von den Schaumelementen senkrecht zur Trägeroberfläche auseinandergedrückt, so daß die Schaumelemente aus den Lücken, in die sie hineinragen, herausgleiten und die Verbundmaterialien sich somit voneinander entfernen.

Die in den nachfolgenden Beispielen beschriebenen Untersuchungen werden wie folgt durchgeführt:

Um die aus dem schaumförmigen Superabsorber extrahierbaren Anteile zu bestimmen, wird eine getrocknete und gemahlene Schaumprobe in einer 0,9 gew.-%igen Kochsalzlösung dispergiert und die Dispersion 1 Stunde gerührt. Danach filtriert man das schaumförmige Material ab und bestimmt die Menge des extrahierten Anteils im Filtrat titrimetrisch.

Die Aufnahmekapazität des Verbundmaterials an Wasser pro Gramm Material wird an Probestücken bestimmt, die ein Gewicht von jeweils 1 g aufweisen. Die Prüfung der Retention erfolgt hierbei nach dem sogenannten Teebeuteltest. Als Flüssigkeit dient dabei eine 0,9 %ige Kochsalzlösung. 1 g des Materials wird in einen Teebeutel gefüllt, der dann verschlossen wird. Dabei ist darauf zu achten, daß der Teebeutel genügend Raum zum vollständigen Ausquellen bietet. Der Teebeutel wird danach eine bestimmte Zeit lang in die Flüssigkeit eingetaucht und nach einer Abtropfdauer von 10 Minuten zurückgewogen. Für die Berechnung der Absorptionskapazität muß ein Blindversuch durchgeführt werden, bei dem ein Teebeutel ohne Material in die Lösung eingetaucht und das Gewicht des Teebeutels nach der oben angegebenen Abtropfdauer von 10 Minuten bestimmt wird. Die Aufnahmekapazität ergibt sich dann aus folgender Beziehung

$$\text{Aufnahmekapazität} = \frac{\text{Gewicht des Teebeutels mit Probe - Gewicht des Teebeutels im Blindversuch}}{\text{Gewicht der eingewogenen Probe}}$$

Die Retention wird folgendermaßen ermittelt:

Gleiche Vorgehensweise wie oben, nur wird anstelle des Abtropfens der Teebeutel 3 min in einer Zentrifuge mit einer Beschleunigung von 250 g geschleudert.

$$\text{Retention} = \frac{\text{Gewicht des Teebeutels nach dem Schleudern - Gewicht des Teebeutels im Blindversuch}}{\text{Gewicht der eingewogenen Probe}}$$

Die Aufnahmegeschwindigkeit (Adsorption Speed, im folgenden mit AS bezeichnet) wurde dadurch ermittelt, daß man aus dem Verbundmaterial bzw. Absorberelement rechteckige Proben mit einem Gewicht von 1 g mit Hilfe eines scharfen Messers ausschnitt. Diese Proben wurden in einer Petrischale mit 20 g synthetischem Urin übergossen. Mit Hilfe einer Stoppuhr wurde die Zeit ermittelt, die die Probe benötigte, um den synthetischen Urin vollständig aufzunehmen. Die Aufnahmegeschwindigkeit (AS) in g/g. sec errechnet sich zu:

$$AS = 20 \text{ g/ } [1 \text{ g} \cdot \text{sec gemessene Zeit (in sec)}]$$

Rezeptur für synthetischen Urin:

In 1 l destilliertem Wasser werden die folgenden Salze gelöst:

| 2,00 g | KCl |
|---|---|
| 2,00 g | $Na_2SO_4$ |
| 0,85 g | $NH_4H_2PO_4$ |
| 0,15 g | $(NH_4)_2HPO_4$ |
| 0,19 g | $CaCl_2$ |
| 0,23 g | $MgCl_2$ |

Die eingesetzten Salze müssen wasserfrei sein.

**Beispiel 1**

In einem Becherglas wurden mit Hilfe eines Magnetrührers die folgenden Komponenten vermischt:

| 224,23 g | einer 37,3 %igen Natriumacrylatlösung in Wasser |
|---|---|
| 49,68 g | Wasser |
| 21,36 g | Acrylsäure |
| 3,15 g | Additionsprodukt von 80 Mol Ethylenoxid an 1 Mol Talgfettalkohol |
| 2,60 g | Pentan |
| 1,05 g | Triacylsäureester eines mit 20 Ethylenoxid veretherten Glycerins |
| 0,53 g | 1,4-Butandioldiacrylat. |

Die erhaltene homogene Mischung wurde in einen 2l-Kolben eingefüllt, in den von unten her Argon eingeleitet wurde. In den Kolben waren zwei Schneebesen eingesetzt, die mit jeweils einem Rührer der Firma Janke & Kunkel Typ RW 20 DZM verbunden waren. Der Argonstrom wurde so eingestellt, daß er mit einer Geschwindigkeit von 80 l/h durch die Reaktionsmischung perlte. Die beiden Rührer wurden zunächst auf eine Drehzahl von 60 UpM eingestellt. Zu der Reaktionsmischung wurden 45,00 g feingemahlener handelsüblicher Superabsorber auf Basis von schwach vernetztem Poly-natriumacrylat (Partikelgröße <100 μm) gegeben und homogen untergemischt. Die freie Öffnung des Kolbens wurde fast vollständig mit Parafilm abgedichtet und die Rührerdrehzahl auf 1000 Upm eingestellt. Die Mischung wurde bei dieser Drehzahl 20 min aufgeschlagen. 5 Minuten vor Ende des Aufschlagprozesses wurden 11,9 g einer 3 %igen, wäßrigen Lösung von 2,2'-Azobis(2-amidinopropan)dihydrochlorid in den Kolben gegeben. Nach Ende der Aufschlagperiode wurde ein feinteiliger, gut fließfähiger Schlagschaum erhalten.

In eine teflonbeschichtete Aluminiumform (Breite: 10 cm, Länge 20 cm) wurde zunächst ein hydrophiliertes, thermobonded Polypropylenvlies (Dicke 0.19 mm, 20 g/m$^2$) eingelegt. Darauf wurde eine 1,5 mm starke Teflonschablone aufgelegt, die 5 mm Bohrungen mit 5 mm Abständen zwischen den Bohrungen entsprechend dem in Fig. 1 skizzierten Raster aufweist. In die Öffnungen wurde der oben herstellte Schaum mit einem Gummiwischer eingestrichen. Dann wurde die Form auf einer konventionellen Heizplatte (Ceran 500) bei einer Heizplattentemperatur von 163°C 2 min erhitzt. Anschließend wurde die Probe 2 min mit einem UV-Strahler (UVASpot 400 der Firma Hönle) bestrahlt, währenddessen die Heizung beibehalten wurde und schließlich wurde die Probe weitere 2 min ohne Bestrahlung erhitzt.

Der nach Abheben der Schablone erhaltene Verbund wurde in einem Vakuumtrockenschrank bei 70°C vollständig getrocknet. Ein Teil der Probe wurde zu Analysenzwecken zermahlen, während der Rest mit destilliertem Wasser auf eine Restfeuchte von 25% eingestellt wurde.

Die auspolymerisierten Schaumelemente hafteten sehr gut am Trägervlies.

Eigenschaften des erhaltenen Verbundmaterials:

Quellungsverhalten:     Die Superabsorberpunkte quollen gleichmäßig unter Erhalt des Verbundes

| Aufnahme | 23.0 g/g |
|---|---|
| Retention | 10.2 g/g |
| Extrahierbare Anteile | 9.8 % |
| AS | 0.5 g/g sec |

**Beispiel 2**

Die Vorgehensweise war identisch mit Beispiel 1, nur wurde als Schablone eine 3 mm starke Teflonschablone mit 8 mm Bohrungen und einem Abstand zwischen den Bohrungen von 11 mm verwendet.

Die auspolymerisierten Schaumelemente hafteten sehr gut am Trägervlies.

Eigenschaften des erhaltenen Verbundmaterials:

Quellungsverhalten: Die Superabsorberpunkte quollen gleichmäßig unter Erhalt des Verbundes

| Aufnahme | 24.10 g/g |
| | 10.8 g/g |
| Extrahierbare Anteile | 9,4 % |
| AS | 0,43 g/g sec |

**Beispiel 3**

Man wiederholte Beispiel 1, wobei jedoch 55,0 g fein gemahlener Superabsorber, wie in Beispiel 1 definiert, verwendet wurden.

Die auspolymerisierten Schaumelemente hafteten sehr gut am Trägervlies.

Eigenschaften des erhaltenen Verbundmaterials:

Quellungsverhalten: Die Superabsorberpunkte quollen gleichmäßig unter Erhalt des Verbundes

| Aufnahme | 22.3 g/g |
| Retention | 9.8 g/g |
| Extrahierbare Anteile | 11,5 % |
| AS | 0.48 g/g sec |

**Vergleichsbeispiel 1**

Es wurde ein Schlagschaum gemäß Beispiel 1 hergestellt.

In eine teflonbeschichtete Aluminiumform (Breite: 10 cm, Länge 20 cm) mit 3 mm hohem Rand wurde dann ein hydrophiliertes, thermobonded Polypropylenvlies (Dicke 0.19 mm, 20 g/m$^2$) eingelegt. Auf dieses Vlies wurde in einer Dicke von 3 mm der obige Schlagschaum gestrichen. Die Form wurde auf einer konventionellen Heizplatte (Ceran 500) bei einer Heizplattentemperatur von 163°C 2 min erhitzt, dann wurde die Probe 2 min mit einem UV-Strahler (UVASpot 400 der Firma Hönle) bestrahlt, währenddessen die Heizung beibehalten wurde und schließlich wurde die Probe weitere 2 min ohne Bestrahlung erhitzt.

Der erhaltene sehr gut aneinander haftende Verbund wurde in einem Vakuumtrockenschrank bei 70°C vollständig getrocknet. Ein Teil der Probe wurde zu Analysenzwecken zermahlen, während der Rest mit destilliertem Wasser auf eine Restfeuchte von 25% eingestellt wurde.

Quellungsverhalten: Beim Aufquellen in einem Überschuß an synthetischem Urin zerrieß die teilgequollene Schaumschicht. Der größere Teil löste sich komplett vom Träger ab, während ein kleiner Teil in Form einer dünnen Schicht auf dem Träger verblieb.

Die abgelöste Schaumschicht quoll isotrop auf.

**Vergleichsbeispiel 2**

Die Schlagschaumherstellung war identisch mit Vergleichsbeispiel 1. In die oben erwähnte Form wurde zunächst ein identisches Vlies wie in Vergleichsbeispiel 1 gelegt. Darauf wurde eine geschlossene Schaumschicht mit 3 mm Dicke gestrichen und die Schaumschicht mit einem weiteren Vlies abgedeckt.

Die Polymerisation erfolgte wie im Vergleichsbeispiel 1.

Es wurde ein Verbund mit sehr gut aneinander haftenden Bestandteilen erhalten.

Quellungsverhalten: Beim Aufquellen in einem Überschuß an synthetischem Urin zerriß der Verbund in 3 Teile. Die teilgequollene Schaumschicht löste sich zum größten Teil von beiden Vliesen ab, während ein kleiner Teil auf den beiden Trägern in Form von dünnen Schichten verblieb. Die abgelösten Schaumschichten quollen isotrop auf.

**Vergleichsbeispiel 3**

Die Schlagschaumherstellung war identisch mit Vergleichsbeispiel 1. Dann wurde eine 1.5 mm hohe Schlagschaumschicht in die oben erläuterte Form eingefüllt und das Vlies eingelegt, auf das eine weitere 1.5 mm hohe Schlagschaumschicht gestrichen wurde. Die weitere Vorgehensweise war wieder identisch mit dem Vergleichsbeispiel 1.

Es wurde wiederum ein Verbund mit sehr gut aneinander haftenden Bestandteilen erhalten.

Quellungsverhalten: Beim Aufquellen in einem Überschuß an synthetischem Urin zerriß der Verbund in 3 Teile. Die teilgequollenen Schaumschichten lösten sich auf beiden Seiten zum größten Teil vom Vlies ab, während ein kleiner Teil auf beiden Seiten des Trägers in Form von dünnen Schichten verblieb. Die abgelösten Schaumschichten quollen isotrop auf.

**Beispiel 4**

Es wurde ein Verbundmaterial wie in Beispiel 1 beschrieben hergestellt.

Aus zwei der erhaltenen Verbundmaterialien wurde durch einfaches Ineinanderschieben in der oben geschilderten Weise ein Absorberelement mit einer Dicke von 2 mm hergestellt. Beim Aufquellen in einem Überschuß von 0.9%iger Kochsalzlösung oder auch von synthetischem Urin trennten sich die beiden ineinander verschachtelten Verbunde. Die Dicke des Absorberelements stieg von 2 mm auf 5.5 mm an.

Eigenschaften des Absorberelements:

| | |
|---|---|
| Aufnahme | 1.96 g/cm$^2$ |
| Retention | 0.88 g/cm$^2$ |
| AS | 0.50 g/g sec |

Die Aufnahmekapazität pro Fläche hat sich gegenüber dem einfachen Verbund in etwa verdoppelt ohne signifikanten Verlust in der Aufnahmegeschwindigkeit.

**Beispiel 5**

Die Vorgehensweise war identisch mit Beispiel 4, nur wurde als Schablone eine Teflonscheibe verwendet, deren Bohrungen keine senkrechten sondern auf 75° abgeschrägte Wände aufwiesen.

Die auspolymerisierten Schaumelemente hafteten sehr gut am Trägervlies.

Eigenschaften des erhaltenen Verbundmaterials:

Quellungsverhalten: Die Superabsorberpunkte quollen gleichmäßig unter Erhalt des Verbundes

| | |
|---|---|
| Aufnahme | 22.1 g/g das entspricht 0.88 g/cm$^2$ |
| Retention | 9.8 g/g das entspricht 0.39 g/cm$^2$ |
| Extrahierbare Anteile | 9.4 % |
| AS | 0.43 g/g sec |

Aus zwei der erhaltenen Verbunde wurde durch einfaches Ineinanderschieben in der oben geschilderten Weise ein Absorberelement mit einer Dicke von 1.9 mm hergestellt. Beim Aufquellen in einem Überschuß von 0.9%iger Kochsalzlösung oder von synthetischem Urin entfernten sich die beiden ineinander verschachtelten Verbunde voneinander, wie in Fig. 4 gezeigt. Die Dicke des Absorberelements stieg von 1.9 mm auf 5.2.

Eigenschaften des Absorberelements:

| Aufnahme | 1.64 g/cm$^2$ |
|---|---|
| Retention | 0.74 g/cm$^2$ |
| AS | 0.45 g/g sec |

Die Aufnahmekapazität pro Fläche hat sich in etwa verdoppelt ohne Verlust in der Aufnahmegeschwindigkeit.

**Patentansprüche**

1. Absorberelement aus wenigstens einem Verbundmaterial mit absorbierenden Elementen auf einem Träger, wobei auf wenigstens einem Träger (3) mehrere Elemente (4) aus einem superabsorbierenden Schaum, die eine Querschnittsfläche im Bereich von 1 bis 400 mm$^2$ aufweisen, rasterförmig in einem solchen Abstand angeordnet sind, der so groß ist wie oder um bis zu 50 % geringer oder um bis zu 100 % größer ist als der Abstand, der erforderlich ist, daß sich die Elemente (4) in gequollenem Zustand an ihren Umfängen berühren, erhältlich durch

   (I) Schäumen einer polymerisierbaren wäßrigen Mischung, die enthält:

   (a) Säuregruppen enthaltende monoethylenisch ungesättigte Monomere, die zu mindestens 50 Mol-% neutralisiert sind,
   (b) Vernetzer,
   (c) wenigstens einen Initiator, und
   (d) 0,1 bis 20 Gew.-% mindestens eines Tensids,

   wobei das Schäumen durch Dispergieren von feinen Blasen eines gegenüber Radikalen inerten Gases erfolgt, und

   (II) Aufbringen der geschäumten Mischung in dem gewünschten Rastermuster auf den Träger, und

   (III) Polymerisieren der geschäumten Mischung unter Bildung eines schaumförmigen Hydrogels und gegebenenfalls Einstellen des Wassergehalts des schaumförmigen Polymerisats auf 1 bis 60 Gew.-%.

2. Absorberelement nach Anspruch 1, **gekennzeichnet durch** zwei Verbundmaterialien (1,2), die einander mit den die Elemente (4) tragenden Seiten so gegenüberliegen, daß die Elemente (4) des einen Verbundmaterials (1) in die Zwischenräume zwischen den Elementen (4) des anderen Verbundmaterials (2) hineinragen.

3. Absorberelement nach Anspruch 1 oder 2, **dadurch gekennzeichnet, daß** bei wenigstens einem Verbundmaterial (1,2) zwischen den gequollenen Schaumelementen (6) Lücken (5) verbleiben.

4. Absorberelement nach Anspruch 3, **dadurch gekennzeichnet, daß** die Schaumelemente (4) Noppen oder noppenartige Erhebungen oder Erhebungen mit fünfeckigem Querschnitt sind.

5. Absorberelement nach Anspruch 1 oder 2, **dadurch gekennzeichnet, daß** die gequollenen Schaumelemente (6) eine geschlossene Oberfläche (7) aufweisen.

6. Absorberelement nach Anspruch 5, **dadurch gekennzeichnet, daß** die Schaumelemente (4) Erhebungen mit dreieckigem, quadratischem, rechteckigem oder sechseckigem Querschnitt sind.

7. Absorberelement nach einem der Ansprüche 1 bis 6, **dadurch gekennzeichnet, daß** der Abstand zwischen zwei Schaumelementen (4) um bis zu 20% geringer ist als der Abstand, der erforderlich ist, damit die gequollenen Schaumelemente (6) miteinander in Berührung kommen.

8. Absorberelement nach einem der Ansprüche 1 bis 6, **dadurch gekennzeichnet, daß** der Abstand zwischen zwei Schaumelementen (4) um bis zu 50% größer ist als der Abstand, der erforderlich ist, damit die gequollenen Schaumelemente (6) miteinander in Berührung kommen.

9. Absorberelement nach einem der vorhergehenden Ansprüche, **dadurch gekennzeichnet, daß** die Schaumelemente (4) eine Höhe von etwa 1 mm bis etwa 20 mm aufweisen.

10. Absorberelement nach einem der vorhergehenden Ansprüche, **dadurch gekennzeichnet, daß** man als Vernetzer (b) eine Mischung aus mindestens einem wasserlöslichen und mindestens einem wasserunlöslichen Vernetzer verwendet.

11. Absorberelement nach Anspruch 10, **dadurch gekennzeichnet, daß** man als Monomere

   (a) Acrylsäure, Methacrylsäure, Vinylsulfonsäure, Acrylamidopropansulfonsäure oder deren Mischungen verwendet.

12. Absorberelement nach Anspruchs 10 oder 11, **dadurch gekennzeichnet, daß** man als wasserlösliche Vernetzer Acrylsäure- und Methacrylsäureester von mindestens zweiwertigen Alkoholen oder Methylenbisacrylamid einsetzt.

13. Absorberelement nach einem der vorhergehenden Ansprüche, **dadurch gekennzeichnet, daß** man in der polymerisierbaren wäßrigen Mischung zusätzlich Verdicker in Form wasserquellbarer oder wasserlöslicher synthetischer oder natürlicher Polymere einsetzt.

14. Absorberelement nach Anspruch 13, **dadurch gekennzeichnet, daß** man als Verdicker pulverförmige Superabsorber einsetzt.

15. Verwendung der Absorberelemente nach einem der Ansprüche 1 bis 14 in Hygiene- oder Sanitärartikeln oder in Wundverbänden.

**Claims**

1. An absorber element of at least one composite material having absorbent elements on a support in which a plurality of. elements (4) of a superabsorbent foam having a cross-sectional area in the range from 1 to 400 mm$^2$ are arranged on at least one support (3) in a grid pattern at a distance which is as great as or up to 50% less than or up to 100% greater than the distance which is required such that the elements (4) in the swollen state touch at their peripheries, obtainable by

   (I) foaming a polymerizable aqueous mixture which consists of

      (a) monoethylenically unsaturated monomers containing acid groups, which are at least 50 mol% neutralized,
      (b) crosslinkers,
      (c) at least one initiator, and
      (d) from 0.1 to 20% by weight of at least one surfactant, the foaming being performed by dispersing fine bubbles of a gas inert to free radicals, and

   (II) applying the foamed mixture to the support in the desired grid pattern, and

   (III) polymerizing the foamed mixture forming a foamed hydrogel and if appropriate setting the water content of the foamed polymer to from 1 to 60% by weight.

2. An absorber element as claimed in claim 1, wherein two composite materials (1, 2) face one another with the sides bearing the elements (4) in a manner such that the elements (4) of the one composite material (1) penetrate into the spaces between the elements (4) of the other composite material (2).

3. An absorber element as claimed in claim 1 or 2, wherein, in the case of at least one composite material (1, 2), gaps (5) remain between the swollen foam elements (6).

4. An absorber element as claimed in claim 3, wherein the foam elements (4) are cylinders or cylindrical elevations or elevations having pentagonal cross section.

**5.** An absorber element as claimed in claim 1 or 2, wherein the swollen foam elements (6) have a closed surface (7).

**6.** An absorber element as claimed in claim 5, wherein the foam elements (4) are elevations having triangular, square, rectangular or hexagonal cross section.

**7.** An absorber element as claimed in one of claims 1 to 6, wherein the distance between two foam elements (4) is up to 20% less than the distance which is required for the swollen foam elements (6) to come into contact with one another.

**8.** An absorber element as claimed in one of claims 1 to 6, wherein the distance between two foam elements (4) is up to 50% greater than the distance which is required for the swollen foam elements (6) to come into contact with one another.

**9.** An absorber element as claimed in one of the preceding claims, wherein the foam elements (4) have a height of from about 1 mm to about 20 mm.

**10.** An absorber element as claimed in one of the preceding claims, wherein, as crosslinker (b), use is made of a mixture of at least one water-soluble and at least one water-insoluble crosslinker.

**11.** An absorber element as claimed in claim 10, wherein, as monomer, use is made of

(a) acrylic acid, methacrylic acid, vinylsulfonic acid, acrylamidopropanesulfonic acid or their mixtures.

**12.** An absorber element as claimed in claim 10 or 11, wherein, as water-soluble crosslinker, use is made of acrylic and methacrylic esters of at least dihydric alcohols, or methylenebisacrylamide.

**13.** An absorber element as claimed in one of the preceding claims, wherein a thickener in the form of water-swellable or water-soluble synthetic or natural polymers is additionally used in the polymerizable aqueous mixture.

**14.** An absorber element as claimed in claim 13, wherein, as thickener, use is made of pulverulent superabsorbents.

**15.** The use of the absorber elements as claimed in one of claims 1 to 14 in hygiene or sanitary articles or in wound dressings.


**Revendications**

**1.** Elément absorbant constitué d'au moins une matière composite qui contient des éléments aptes à l'absorption sur un support, **caractérisé en ce que** plusieurs éléments (4) constitués de mousse superabsorbante et présentant une superficie de section comprise entre 1 mm$^2$ et 400 mm$^2$, sont disposés sur au moins un support (3), en formant une trame et à une distance qui, par rapport à la distance permettant le contact des éléments (4) au niveau de leurs circonférences quand ils sont gonflés, est identique ou inférieure jusqu'à 50% ou supérieure jusqu'à 100%, et que l'on peut obtenir par

(I) une expansion d'un mélange aqueux polymérisable qui contient :

(a) des monomères à insaturation monoéthylénique qui présentent des groupes acide et qui sont neutralisés à raison d'au moins 50% en moles,
(b) des agents de réticulation,
(c) au moins un amorceur et
(d) au moins un agent tensioactif à raison de 0,1% à 20% en poids,

où on réalise l'expansion au moyen d'une dispersion, sous forme de bulles fines, d'un gaz qui est inerte par rapport aux radicaux et
(II) une application du mélange expansé sur le support, en formant la trame choisie, et
(III) une polymérisation du mélange mousseux en obtenant un hydrogel sous forme de mousse et, éventuellement, en réglant la teneur d'eau du polymère sous forme de mousse à une teneur comprise entre 1% et 60% en poids.

2. Elément absorbant selon la revendication 1, **caractérisé par** deux matières composites (1, 2) qui sont disposées de manière que leurs cotés portant les éléments (4) sont situés l'un face à l'autre, de telle sorte que les éléments (4) de la première matière composite (1) font saillie dans les interstices entre les éléments (4) de l'autre matière composite (2).

3. Elément absorbant selon la revendication 1 ou 2, **caractérisé en ce que** des creux (5) subsistent entre les éléments mousse (6) gonflés, pour au moins une des matières composites (1, 2).

4. Elément absorbant selon la revendication 3, **caractérisé en ce que** les éléments mousse (4) forment ou des nopes ou des proéminences ayant une forme de nope ou des proéminences à section pentagonale.

5. Elément absorbant selon la revendication 1 ou 2, **caractérisé en ce que** les éléments mousse gonflés (6) présentent une surface (7) lisse.

6. Elément absorbant selon la revendication 5, **caractérisé en ce que** les éléments mousse (4) sont des proéminences à section triangulaire, carrée, rectangulaire ou hexagonale.

7. Elément absorbant selon une des revendications 1 à 6, **caractérisé en ce que** l'espacement entre deux éléments mousse (4) est inférieur, à raison de jusqu'à 20%, par rapport à l'espacement nécessaire pour que les éléments mousse gonflés (6) rentrent mutuellement en contact.

8. Elément absorbant selon une des revendications 1 à 6, **caractérisé en ce que** l'espacement entre deux éléments mousse (4) est supérieur, à raison de jusqu'à 50%, par rapport à l'espacement nécessaire pour que les éléments mousse gonflés (6) rentrent mutuellement en contact.

9. Elément absorbant selon une quelconque des revendications précédentes, **caractérisé en ce que** les éléments mousse (4) présentent une hauteur comprise entre environ 1 mm et environ 20 mm.

10. Elément absorbant selon une quelconque des revendications précédentes, **caractérisé en ce que** l'on met en oeuvre, en tant qu'agent de réticulation (b), un mélange constitué d'au moins un agent de réticulation soluble dans l'eau et d'au moins un agent de réticulation non soluble dans l'eau.

11. Elément absorbant selon la revendication 10, **caractérisé en ce que** l'on met en oeuvre, en tant que monomères,

    (a) l'acide acrylique, l'acide méthacrylique, l'acide vinylsulfonique, l'acide acrylaminopropansulfonique ou leurs mélanges.

12. Elément absorbant selon la revendication 10 ou 11, **caractérisé en ce que** l'on met en oeuvre, en tant qu'agent de réticulation soluble dans l'eau, les esters de l'acide acrylique et de l'acide méthacrylique d'alcools qui sont au moins bivalents, ou bien la méthylène-bisacrylamide.

13. Elément absorbant selon une quelconque des revendications précédentes, **caractérisé en ce que** l'on met de plus en oeuvre, dans le mélange aqueux polymérisable, des agents épaississants sous forme de polymères naturels ou synthétiques, solubles dans l'eau ou gonflables dans l'eau.

14. Elément absorbant selon la revendication 13, **caractérisé en ce que** l'on met en oeuvre, en tant qu'agents épaississants, des agents superabsorbants sous forme de poudre.

15. Mise en oeuvre des éléments absorbants selon une quelconque des revendications 1 à 14 dans les produits d'hygiène ou de santé ou dans les pansements.

Fig. 1

Fig. 2

Fig. 3

EP 0 884 993 B1

Fig. 4